Europäisches Patentamt

European Patent Office

Office européen des brevets

(11) Publication number: **0 166 232**
**A2**

(12) **EUROPEAN PATENT APPLICATION**

(21) Application number: **85106445.1**

(22) Date of filing: **24.05.85**

(51) Int. Cl.⁴: **A 61 K 7/08**

(30) Priority: **30.05.84 US 615181**

(43) Date of publication of application: **02.01.86**
**Bulletin 86/1**

(84) Designated Contracting States: **AT BE CH DE FR GB IT LI LU NL SE**

(71) Applicant: **S.C. JOHNSON & SON, INC., 1525 Howe Street, Racine, Wisconsin 53403 (US)**

(72) Inventor: **Sramek, John A., 4 Redwood Court, Racine Wisconsin (US)**

(74) Representative: **Baille, Iain Cameron et al, c/o Ladas & Parry Isartorplatz 5, D-8000 München 2 (DE)**

(54) Foaming hair cleanser and conditioner.

(57) An aqueous lathering conditioning cleanser includes a neutralized cationic foaming surfactant and an emulsified conditioning system of a cationic emulsifying conditioner and water insoluble emulsifiable conditioning agent.

EP 0 166 232 A2

- 1 -

The present invention relates to a novel hair cleansing and conditioning composition with lathering action.  More particularly it relates to a lathering composition which is capable of cleansing and conditioning the hair while leaving little or no residue.

Shampoos and their method of use are well-known, Their purpose is to clean the hair by removing soil.  However, they also remove natural fats and oils.  Shampoos often leave hair tangled and unmanageable due to their drying nature and often promote "fly away" due to static build up.

Conditioners were developed in response to the problems created by shampoos.  Most conditioners contain cationic agents which lubricate, moisturize and/or soften the hair.  Conditioners are generally applied after shampooing is complete, and they must be rinsed from the hair.

Many beneficial results are gained by the use of a conditioner, including reduction in static build up and "fly-away", improved manageability, resiliency and body, prevention of damage to hair from hair appliances such as dryers and hot curlers, the ability to hold hair in a preset configuration and improved control, shine, texture and feel of the hair.

A major disadvantage of conditioner use is that they often build up on the hair or leave behind a residue, making the user feel the hair is not completely clean.  A second major disadvantage is that a two step process is needed to first cleanse and then condition hair.  This is wasteful of both time and money.

Attempts to make stable and effective conditioning shampos have not been satisfactory and have included adding a variety of different ingredients to shampoo formulations, such as vitamins, protein, mink oil, placenta, lanolin, silicones and cationic surfactants.  It has also been proposed to combine certain anionic detergents with compatible cationic surfactants in an attempt to provide a conditioning shampoo which simultaneously cleans and conditions.  However, to date, such shampoos have not provided satisfactory balanced performance in terms of cleaning and conditioning.  Examples of such proposed formulations are found in U. S. Patent Nos. 4,132,678; 4,168,302; 4,220,548 and 4,330,526.

Lathering cleanser conditioners have been developed.  Most of these formulations, however, contain both anionic and cationic surfactants.  Such surfactant combinations tend to react in the presence of the ambient oil and dirt on the hair to form a residue which leaves hair feeling unclean.

U. S. Patent No. 4,333,921 discloses a lathering conditioner which is said to leave the hair feeling clean, but which has been found to be an ineffective cleanser. This formulation contains only cationic ingredients.  An alkyl dimethylamine oxide (lauryldimethyl amine oxide) was proposed as a foam booster in conjunction with a known cationic conditioner, such as alkyl dimethyl benzylammonium chloride.  The amine oxide is said to penetrate the hair and to leave the hair clean feeling.  This system suffers from a variety of defects, including an inability to rinse clean.  The system also leaves behind an unacceptable residue on the hair, which can cause foaming during wet combing.

Amine oxides are commercially supplied with some residual free hydrogen peroxide which can cause serious formulation and application (oxidation) problems.  Amine oxides are also unstable at high temperatures, at pH's above 6 and in the presence of metal ions.

Accordingly, an acute need has arisen for a stable, effective formulation for effectively cleaning and conditioning the hair. Such a shampoo must be safe and nontoxic and must impart to the hair: improved wet and dry combing properties; improved manageability (freedom from tangling during grooming); enhanced body (improved strength and resiliency); the ability to efficiently retain curls or waves; freedom from static charge buildup; and enhanced luster, (reflection of light scattered by the hair) feel and softness. Until now, proposed conditioning shampoos have lacked such balanced performance. Improvement in either cleansing or conditioning has been at the sacrifice of the other.

The object of the present invention is to provide a safe, lathering hair cleanser and conditioner product which rinses clean and leaves little or not discernable residues on the hair.

The present invention provides an aqueous lathering conditioning cleanser having an acidic pH comprising a cationic conditioner and a surfactant characterized by:

    a. an emulsified conditioning system including:

        i) a cationic emulsifying conditioner, and

        ii) a water insoluble emulsifiable conditioning agent; and

    b. a neutralized cationic foaming surfactant; wherein the emulsified conditioning system is less water soluble than the neutralized cationic foaming surfactant.

The lathering product of the present invention aids in the dispersal of the conditioner through the hair and has good dry and wet combability and sensitivity.

The product of the present invention is capable of both cleansing and conditioning the hair, leaving it clean, manageable and with good cosmetic qualities.

The composition may also include, for example, an acidifying agent to maintain the pH in an acid range of, 2.5 to 6.5, preferably 3 to 5; a silicone oil to further reduce foam residue on the hair, and, a thickener.

The composition may also include, for example, an

- 4 -                    0166232

acidifying agent to maintain the pH in an acid range of,
2.5 to 6.5, preferably 3 to 5; a silicone oil to further
reduce foam residue on the hair, and, a thickener.

The cationic emulsifying conditioner of the
invention includes a quaternary surfactant including mono-
fatty di-loweralkyl benzyl quaternary surfactants, mono-
fatty tri-lower alkyl quaternary surfactants, di-fatty di-
lower alkyl quaternary surfactants, and di-fatty di-lower
alkyl diquaternary surfactants. Examples of suitable
cationic emulsifying conditioners include distearyl dimethyl
ammonium chloride, stearyl dimethyl benzyl ammonium
chloride, cetyl trimethyl ammonium-chloride, dicetyl dimethyl ammonium chloride, and Jordaquat Dimer 18 (bis/stearyl
dimethyl ammonium) hydroxy-propyl dichloride (supplied by
Jordan Chemical Co.).

The cationic emulsifying conditioner also includes
acid salts of fatty amines and fatty amido amines, wherein
the fatty group is $C_{16}$ to $C_{20}$. Typical fatty amine salts
and fatty amide salts include stearyl dimethyl amine
hydrochloride, stearyl amido propyl dimethylamine hydrochloride, and cetyl dimethyl amine citrate. A preferred
amine is stearyl dimethyl amine. The amines are acidified
with conventional inorganic or typical organic and
carboxylic acids, including hydrochloric acids, phosphoric
acid and preferable citric acid.

Emulsifying conditioners with an equivalent weight
below about 300 are generally too water soluble for the
purposes of the present invention and usually do not make
stable emulsions and/or do not condition the hair. Emulsifying conditioners with an equivalent weight about 600 often
fail to emulsify properly in the system of the present
invention. Accordingly, it is preferred to employ a
cationic emulsifying conditioner with an equivalent weight
from about 300 to 600.

The water-insoluble emulsifiable conditioning agents of the present invention which are the second ingredient in the emulsified conditioning system should have the ability to separate from water and form a two-phase composition with it, when it is used alone. For this purpose, the water-insoluble emulsifiable conditioning agents are preferably less than one percent soluble in water. Such agents may be employed either as liquids or as solids. Examples of such agents include $C_{14}$ to $C_{22}$ fatty alcohols, preferably cetyl alcohol; fatty esters of $C_{12}$ to $C_{18}$ acids and $C_2$ to $C_{18}$ alcohols where the total number of carbon atoms is from about 16 to about 36 and esters of lower alkyl polyols, such as ethylene glycol, glycerine, diethylene glycol and triethylene glycol with $C_{12}$ to $C_{20}$ fatty acids. Mineral oils can also be used. Additionally, mixtures of said water-insoluble conditioning agents may be used.

The cationic emulsifying conditioner and the water-insoluble, emulsifyable conditioning agent of the invention are employed in a combined amount of from about 0.5% to about 10% based upon the total weight of the aqueous composition. Use of about 2% of the emulsifying conditioner and emulsifiable conditioning agent is preferred. The weight ratio of emulsifying conditioner to emulsifiable conditioning agent may range from 5:1 to 1:5, with 1.5 to 1 being the preferred ratio.

The cationic foaming surfactant of the invention includes alkyl amido dimethyl $C_{10}$ to $C_{18}$ amines such as lauryl amidopropyl dimethylamine, cocoamidopropyl dimethyl amine and $C_{10}$ to $C_{18}$ alkyl dimethyl amines such as myristyl dimethylamine and cetyl dimethyl amine. The cationic foaming surfactant of this invention is neutralized to a pH of 2.5 to 8.0 before use with a suitable organic or inorganic acid, preferably a carboxylic acid, such as citric acid.

If desired, amine oxides and $C_{10}$ to $C_{18}$ amidoamine oxides, such as laurylamine oxide and cocoamidoamine oxide, may also be used as the cationic foaming surfactant, if the known disadvantages of such compounds are taken into account during formulation and use.

The foaming conditioning cleanser of the invention contains preferably from about 0.2% to about 20% of the cationic foaming surfactant. If less than 0.2% of the foaming surfactant is used, there is little or no perceivable foaming in the product. Amounts greater than 20% of the cationic foaming surfactant produce unnecessary cleaning and are unduly costly to formulate.

To produce the foaming conditioner and cleanser of the invention, the cationic emulsifying conditioner and the water insoluble emulsifiable conditioning agent are first dispersed in water to produce an emulsion. The resulting emulsion should be less water soluble than the cationic foaming surfactant, so that the emulsion is not itself emulsified by the cationic foaming surfactant. If the cationic foaming surfactant emulsifies the conditioning emulsion, the emulsion will be rinsed from the hair. The conditioners must coat the hair shaft to provide proper conditioning.

Accordingly, the preparation temperatures for the conditioning cleanser of the invention must be sufficiently low to prevent emulsification of the preformed emulsion when all the ingredients are combined. For this purpose the formulation temperature is usually less than about 130°F. When the conditioning emulsion is less water soluble than the cationic foaming surfactant, a conditioning system is deposited on the hair which provides excellent wet combing, dry combing and conditioning properties.

The pH of the lathering conditioner cleanser of the invention should be adjusted from about 2.5 to 6.5 to neutralize the basic amine functionality in the cationic foaming surfactant. A final composition pH of 3 to 6 is preferred. Any nontoxic, compatible acid may be used to accomplish this purpose. Citric acid and phosphoric acid are preferred.

Other additives and adjuvents may also be included in the composition of the invention. Thickening agents may be employed to control the viscosity of the formulation. Preferred thickeners are hydrophilic polymers, hydroxymethyl cellulose and hydroxpropyl cellulose. Other suitable additives include fragrances, dyes, preservatives, cationic polymers, amphoteric polymers, proteins, amino acids, nonionic surfactants, pearlizing agents, salts for thickening and the like.

The following examples illustrate certain preferred embodiments of the invention and are not intended to limit the scope of the present invention. The weights are based on the total weight of the aqueous composition.

### Example 1

A lathering conditioner of the invention is prepared as follows:

A pre-formed emulsion was first prepared from distearyl dimethyl ammonium chloride, cetyl alcohol, silicone fluid and water. This emulsion was then added to the remaining ingredients at a temperature of 90°F to keep the emulsion from being emulsified by the cationic surfactant. The final solution pH was adjusted to 3.3. The cleanser had the following composition:

| Ingredient | Amount |
|---|---|
| Distearyl dimethyl ammonium chloride | 1.20 |
| Cetyl alcohol | 0.80 |
| Dimethyl silicone fluid (500 centistokes) | 0.30 |
| Laurylamido propyl dimethyl amine | 1.80 |
| Stearyl dimethyl amine | 0.20 |
| Hydroxypropyl methyl cellulose | 1.50 |
| Dye solution | 0.25 |
| Fragrance | 0.35 |
| Potassium hydroxide | 0.005 |
| Citric acid | 1.10 |
| Water | 92.495 |
| Total | 100.000 |

The silicone fluid employed in the formulation tends to reduce foam residue on the hair after rinsing.

In order to demonstrate the superior properties of the present invention a comparative test was run. Three formulations of the present invention were prepared according to the procedure of Example 1 and were tested against a prior art composition of U.S. Patent No. 4,333,921. The results clearly demonstrate that the compositions of the invention show improved properties in respect of reduction of both the dry combing force and residue on the comb after rinsing. The three formulations were as follows:

| Formula 1 | Amount |
|---|---|
| Stearyl dimethyl amine | 0.2 |
| Cetyl Alcohol | 0.8 |
| Laurylamido propyl dimethyl amine | 1.8 |
| Distearyldimethyl ammonium chloride | 1.2 |
| Methylisothiazolinone | 0.03 |
| Hydroxypropylmethyl cellulose | 1.1 |
| Citric Acid | 1.1 |
| Fragrance | 0.3 |
| Dye | 0.2 |
| Water | 93.27 |

Formula 2 was the same as formula 1 except that cocoamidoamine oxide was substituted for laurylamidopropyl dimethyl amine. In formula 3 laurylamine oxide was substituted for the laurylamido propyl dimethyl amine.

A composition of United States Patent No. 4,333,921 was prepared as follows:

PRIOR ART COMPOSITION

| Ingredient | Amount |
|---|---|
| Lauramine oxide - 30% | 13.3 |
| stearyl dimethyl benzyl ammonium chloride | 1.5 |
| Glycerin | 5.0 |
| Sorbitan oleate | 2.5 |
| Hydroxy ethylcellulose | 1.0 |
| Citric acid | 0.6 |
| Glycol stearate | 0.5 |
| Cetrimonium chloride - 25% | 0.25 |
| Fragrance | 1.0 |
| Water | 74.35 |

This composition was prepared by adding the hydroxyethylcellulose to the water with mixing, and heating to 110°F. Mixing was continued for 20 minutes. The citric acid, glycol stearate, cetrimonium chloride, stearyl dimethyl benzyl ammonium chloride, lauramine oxide, glycerin and sorbitan oleate were then added. The mixture was heated to 150°F with stirring, until the mix was uniform. The mixture was then cooled to 110°F, and the fragrance added. The final composition was cooled to room temperature with stirring.

The three formulas of the present invention and the composition of the '921 patent were then applied to swatches of human hair and agitated until foaming occurred. The products were then thoroughly rinsed from the hair.

The results of these tests are shown below.

0166232

| Property | Formula 1 | Formula 2 | Formula 3 | '921 Patent |
|---|---|---|---|---|
| Foaming | Very good | Very good+ | Very good+ | Very good+ |
| Wet combing | Very good+ | Good- | Good+ | Good+ |
| Residue on comb (foam) | Some<br>1 cc | Some+<br>2 cc | Some+<br>2 cc | a lot<br>5-10cc |
| Dry combing force | 39.6g | 35.9g | 51.5g | 100.9g |

The residue on comb (foam) as reported in the comparative results was for an 8" comb. The superiority of the claimed foaming conditioners in reducing residue remaining on the hair and the amount of dry combing force required to manage the hair after treatment over the prior art composition is clearly shown by the test results.

The invention is not to be limited except as set forth in the following claims.

CLAIMS

1. An aqueous lathering conditioning cleanser having an acidic pH comprising a cationic conditioner and a surfactant characterized by:

    a. an emulsified conditioning system including;

        i) a cationic emulsifying conditioner, and

        ii) a water insoluble emulsifiable conditioning agent; and

    b. a neutralized cationic foaming surfactant;

wherein the emulsified conditioning system is less water soluble than the neutralized cationic foaming surfactant.

2. The composition according to claim 1 characterized in that the cationic emulsifying conditioner is selected from the group consisting of a quaternary surfactant having an equivalent weight from about 300 to 600 and an acid salt of a fatty amine or fatty amido amine.

3. The composition according to claim 1 or 2 characterized in that the water insoluble emulsifying conditioning agent is selected from the group consisting of $C_{14}$ to $C_{22}$ fatty alcohols; fatty esters of $C_2$ to $C_{18}$ acids and $C_2$ to $C_8$ alcohols wherein the total number of carbon atoms is from 16 to 36; esters of $C_{12}$ to $C_{20}$ fatty acids; and mineral oils.

4. The composition according to claim 1, 2 or 3 characterized in that the cationic foaming surfactant is selected from the group consisting of a $C_{10}$ to $C_{18}$ alkyl amido dimethyl amines and salts thereof; $C_{10}$ to $C_{18}$ alkyl dimethyl amines and salts thereof; $C_{10}$ to $C_{18}$ amine oxides; and $C_{10}$ to $C_{18}$ amidoamine oxides.

5. The composition according to any of claims 1 to 4 characterized in that the cationic emulsifying conditioner is distearyl dimethyl ammonium chloride.

6. The composition according to any of claims 1 to 5 characterized in that the water insoluble conditioning agent is cetyl alcohol.

7. The composition according to any of claims 1 to 6 characterized in that the cationic foaming surfactant is laurylamido propyl dimethyl amine.

8. An aqueous lathering conditioning cleanser composition having a pH from about 2.5 to 7.0 characterized by:

    a. from 0.5 to 10% by weight of said composition of an emulsion including:

        i) a cationic emulsifying conditioner; and

        ii) a water insoluble emulsifiable conditioning agent, the weight ratio of i) and ii) being from 1 to 5 to 5 to 1; and

    b. from 0.05 to 20% by weight of said composition of a neutralized cationic foaming surfactant,

wherein the emulsion (a) is less water soluble than the neutralized cationic foaming surfactant.

9. The composition according to any of claims 1 to 8 characterized by including a thickener.

10. The composition according to any of claims 1 to 9 characterized by containing from about 0.1 to 0.5% by weight of said composition of silicone fluid.